(19) Europäisches Patentamt
European Patent Office
Office européen des brevets

(11) EP 3 973 963 A1

(12) EUROPEAN PATENT APPLICATION
published in accordance with Art. 153(4) EPC

(43) Date of publication:
**30.03.2022 Bulletin 2022/13**

(21) Application number: **20808747.8**

(22) Date of filing: **25.05.2020**

(51) International Patent Classification (IPC):
***A61K 31/4709*** *(2006.01)* ***A61P 35/00*** *(2006.01)*
***A61P 35/04*** *(2006.01)*

(52) Cooperative Patent Classification (CPC):
**A61K 31/4709; A61P 35/00; A61P 35/04**

(86) International application number:
**PCT/CN2020/092125**

(87) International publication number:
**WO 2020/233723 (26.11.2020 Gazette 2020/48)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **23.05.2019 CN 201910433564**

(71) Applicant: **Chia Tai Tianqing Pharmaceutical Group Co., Ltd.**
**Lianyungang City, Jiangsu 222062 (CN)**

(72) Inventors:
- **WANG, Shanchun**
**Lianyungang City, Jiangsu 222062 (CN)**
- **ZHANG, Bin**
**Beijing 100036 (CN)**
- **WANG, Weifeng**
**Haikou City, Hainan 570208 (CN)**
- **XU, Ping**
**Lianyungang City, Jiangsu 222062 (CN)**

(74) Representative: **v. Bezold & Partner Patentanwälte - PartG mbB**
**Akademiestraße 7**
**80799 München (DE)**

# (54) QUINOLINE DERIVATIVES FOR TREATMENT OF HEAD AND NECK CANCER

(57) Provided is an application of a compound, 1-[[[4-(4-fluoro-2-methyl-1H-indol-5-yl)oxy-6-methoxyquinolin-7-yl] oxy]methyl]cyclopropylamine, in preparation of a medicament for the treatment of head and neck cancer, and an application of a pharmaceutical composition of the compound combined with a second therapeutic drug in the preparation of a medicament for the treatment of head and neck cancer. The head and neck cancer can also be nasopharyngeal cancer.



Printed by Jouve, 75001 PARIS (FR)

## Description

## TECHNICAL FIELD

The present application belongs to the technical field of pharmaceuticals, and relates to use of a quinoline derivative for combating tumors. In particular, the present application relates to a quinoline derivative for use in treating head and neck cancer, a pharmaceutical composition thereof in combination with a second therapeutic agent and use thereof for treating head and neck cancer.

## BACKGROUND

Head and neck cancer refers to other malignancies, except brain cancer, located in the head and neck region. More commonly, it is oral cancer, nasopharyngeal carcinoma, oropharyngeal cancer, hypopharyngeal cancer, laryngeal cancer, sinus cancer, salivary gland cancer or the like. Head and neck cancer commonly occurs in the oral cavity, nasal cavity, throat, sinus, salivary gland, larynx and the like. The symptoms of head and neck cancer include a sore or a lump that does not heal, a sore throat that does not go away, difficulty in swallowing, and a change in the voice. What's more, the symptoms of head and neck cancer may further include unusual bleeding, facial swelling and dyspnea.

In 2015, 5.5 million people all over the world suffer from head and neck cancer (2.4 million in the oral cavity, 1.7 million in the throat and 1.4 million in the larynx), and more than 379,000 people die from the disease (146,000 from oral cancer, 127,400 from throat cancer and 105,900 from laryngeal cancer). Head and neck cancer is considered as the seventh most common cancer worldwide and the ninth most common cause of death worldwide. About 1% of people in the United States have suffered from head and neck cancer, with the number of male patients twice as many as those of female patients and the usual age at diagnosis between 55 and 65 years old. In developed countries, the average five-year survival rate following diagnosis is 42%-64%.

Nasopharyngeal carcinoma is a malignant tumor occurring in the roof and lateral walls of the nasopharyngeal cavity, which is one of the high-incidence malignant tumors in China, and has the highest incidence rate among the malignant tumors in the ears, nose, pharynx and larynx. The World Health Organization (WHO) has classified nasopharyngeal carcinoma in the following major types (2005 edition): type I keratinizing squamous cell carcinoma and type II non-keratinizing squamous cell carcinoma, and the latter is classified into a differentiated type and an undifferentiated type. Epidemiological data show that, 98% of patients diagnosed with nasopharyngeal carcinoma in high-incidence areas are type II patients, and only 2% are type I patients. The 5-year survival status of patients diagnosed with type I and type II nasopharyngeal carcinoma is remarkably different, while the 5-year survival status of patients diagnosed with differentiated and undifferentiated types in type II is not remarkably different. The nasopharyngeal carcinoma shows diversity in pathological morphology, and has no significant relevance with clinical significance.

Chen Size et al in "study on the diagnosis and role of NLK expression in nasopharyngeal carcinoma" pointed out that nasopharyngeal carcinoma mostly has moderate sensitivity to radiation therapy, which is the first choice for the treatment of nasopharyngeal carcinoma. However, surgical resection and chemotherapy are indispensable for treating patients having well-differentiated cancer, relatively advanced cancer, and a relapse after radiotherapy. The complicated nasopharyngeal structure, and the deep and hidden diseased regions make it difficult to treat the disease. Besides, individual specific treatment and medicaments are lacking, resulting in treatment lacking variety and thus still poor treatment effect on nasopharyngeal carcinoma and always low 5-year survival rates.

## BRIEF SUMMARY

In a first aspect, the present application provides use of a compound I or a pharmaceutically acceptable salt thereof for preparing a medicament for use in treating head and neck cancer. The present application further provides a method for treating head and neck cancer, comprising administering to a subject a compound I or a pharmaceutically acceptable salt thereof. The present application further provides use of a compound I or a pharmaceutically acceptable salt thereof for treating head and neck cancer.

In a second aspect, the present application provides a combined pharmaceutical composition for use in treating head and neck cancer, comprising: (i) a compound I or a pharmaceutically acceptable salt thereof; and (ii) at least one second therapeutic agent.

In a third aspect, the present application further provides use of a pharmaceutical composition for preparing a medicament for use in treating head and neck cancer. The present application further provides use of a pharmaceutical composition for treating head and neck cancer.

In a fourth aspect, the present application further provides a method for treating head and neck cancer, comprising administering to a subject the pharmaceutical composition disclosed herein. The pharmaceutical composition comprises: (i) a compound I or a pharmaceutically acceptable salt thereof; and (ii) at least one second therapeutic agent.

SUMMARY

In a first aspect, the present application provides use of a compound I or a pharmaceutically acceptable salt thereof for preparing a medicament for use in treating head and neck cancer,

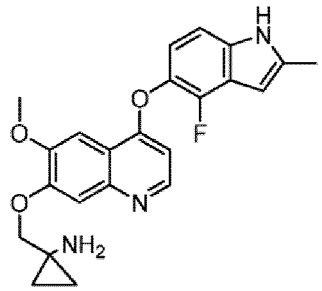

compound I.

In some embodiments, the head and neck cancer is head and neck squamous cell carcinoma.

In some embodiments, the head and neck cancer is head and neck adenocarcinoma.

In some embodiments, the head and neck cancer includes, but is not limited to, neck tumors, otorhinolaryngological tumors and oromaxillofacial tumors. In some embodiments, the head and neck cancer includes, but is not limited to, oral cancer, laryngeal cancer, parotid gland cancer, nasal cavity cancer, sinus cancer, paranasal sinus cancer, oropharyngeal cancer, floor of mouth cancer, hypopharyngeal cancer, palate cancer, gum cancer, tongue cancer, tongue mucosal squamous cell carcinoma, buccal mucosal carcinoma, lip cancer, salivary gland cancer, tonsil cancer, and the like.

In some embodiments, the head and neck adenocarcinoma, according to its histopathological analysis, includes, but is not limited to, mucoepidermoid carcinoma, adenoid cystic carcinoma, lymphoepithelial carcinoma, pleomorphic adenocarcinoma, salivary duct carcinoma, myoepithelial carcinoma, non-specific adenocarcinoma, non-specific clear cell carcinoma, cystadenocarcinoma, sebaceous adenocarcinoma, sebaceous lymphoid adenocarcinoma, mucinous adenocarcinoma, and epithelial-myoepithelial carcinoma.

In a second aspect, the present application provides a combined pharmaceutical composition for use in treating head and neck cancer, comprising (I) a compound I or a pharmaceutically acceptable salt thereof; and (ii) at least one second therapeutic agent.

In some embodiments of the present application, the combined pharmaceutical composition comprises: (i) a pharmaceutical composition of the compound I or the pharmaceutically acceptable salt thereof; and (ii) a pharmaceutical composition of the at least one second therapeutic agent. In some embodiments, provided is a combined pharmaceutical composition for use in treating head and neck cancer, comprising: (i) the compound I or the pharmaceutically acceptable salt thereof; and (ii) at least one chemotherapeutic agent, optionally in combination with radiation therapy. In some embodiments, provided is a combined pharmaceutical composition for use in treating head and neck cancer, comprising: (i) the compound I or the pharmaceutically acceptable salt thereof; and (ii) at least one small molecule targeted anti-tumor agent, optionally in combination with radiation therapy. In some embodiments, provided is a combined pharmaceutical composition for use in treating head and neck cancer, comprising: (i) the compound I or the pharmaceutically acceptable salt thereof; and (ii) at least one immunotherapeutic agent, optionally in combination with radiation therapy. In some embodiments, provided is a combined pharmaceutical composition for use in treating head and neck cancer, comprising: (i) the compound I or the pharmaceutically acceptable salt thereof; and (ii) at least one macromolecular antibody drug, optionally in combination with radiation therapy.

In some embodiments, provided is a combined pharmaceutical composition for use in treating head and neck cancer, comprising: (i) the compound I or the pharmaceutically acceptable salt thereof; and (ii) a platinum agent, optionally in combination with radiation therapy. In some embodiments, provided is a combined pharmaceutical composition for use in treating head and neck cancer, comprising: (i) the compound I or the pharmaceutically acceptable salt thereof; and (ii) a platinum agent and at least one selected from the group consisting of a fluoropyrimidine derivative and an anti-PD-1 antibody, optionally in combination with radiation therapy. In some embodiments, provided is a combined pharmaceutical composition for use in treating head and neck cancer, comprising: (i) the compound I or the pharmaceutically acceptable salt thereof; and (ii) a platinum agent and cotuximab, optionally in combination with radiation therapy. In some embodiments, provided is a combined pharmaceutical composition for use in treating head and neck cancer, comprising: (i) the compound I or the pharmaceutically acceptable salt thereof; and (ii) gemcitabine and a platinum agent, optionally in combination with radiation therapy. In some embodiments, provided is a combined pharmaceutical composition for use in treating head and neck cancer, comprising: (i) the compound I or the pharmaceutically acceptable salt thereof; and (ii) gemcitabine and cisplatin, optionally in combination with radiation therapy. In some embodiments,

provided is a combined pharmaceutical composition for use in treating head and neck cancer, comprising: (i) the compound I or the pharmaceutically acceptable salt thereof; and (ii) at least one selected from the group consisting of a fluoropyrimidine derivative and taxanes, optionally in combination with radiation therapy. In some embodiments, provided is a combined pharmaceutical composition for use in treating head and neck cancer, comprising: (i) the compound I or the pharmaceutically acceptable salt thereof; and (ii) gemcitabine and paclitaxel, optionally in combination with radiation therapy. In some embodiments, provided is a combined pharmaceutical composition for use in treating head and neck cancer, comprising: (i) the compound I or the pharmaceutically acceptable salt thereof; and (ii) cisplatin and 5-fluorouracil, optionally in combination with radiation therapy. In some embodiments, provided is a combined pharmaceutical composition for use in treating head and neck cancer, comprising: (i) the compound I or the pharmaceutically acceptable salt thereof; and (ii) sintilimab, optionally in combination with radiation therapy. In some embodiments, provided is a combined pharmaceutical composition for use in treating head and neck cancer, comprising: (i) the compound I or the pharmaceutically acceptable salt thereof; and (ii) capecitabine, optionally in combination with radiation therapy.

In a third aspect, the present application provides use of a pharmaceutical composition for preparing a medicament for use in treating head and neck cancer, comprising: (i) a compound I or a pharmaceutically acceptable salt thereof; and (ii) at least one second therapeutic agent, optionally in combination with radiation therapy.

In a fourth aspect, the present application further provides a method for treating head and neck cancer, comprising administering to a subject the pharmaceutical composition disclosed herein. The pharmaceutical composition comprises: (i) a compound I or a pharmaceutically acceptable salt thereof; and (ii) at least one second therapeutic agent.

The present application provides a method for treating a patient with head and neck cancer. In some embodiments of the present application, the patient has previously received surgical treatment, chemotherapy, and/or radiation therapy. In some specific embodiments, progressive disease recurs after the patient has achieved complete response following surgical treatment, chemotherapy, and/or radiation therapy. In some specific embodiments, the patient has failed to achieve complete response or partial response following surgical treatment, chemotherapy and/or radiation therapy.

The present application provides a method for treating head and neck cancer, comprising administering to a patient in need thereof a compound I or a pharmaceutically acceptable salt thereof, and at least one second therapeutic agent. In some embodiments, the present application provides a method for treating head and neck cancer that has not been treated with chemotherapeutic drugs, comprising administering to a patient in need thereof the compound I or the pharmaceutically acceptable salt thereof, and the at least one second therapeutic agent. In some embodiments, the present application provides a method for treating head and neck cancer that has been treated with at least one chemotherapeutic drug, comprising administering to a patient in need thereof the compound I or the pharmaceutically acceptable salt thereof, and the at least one second therapeutic agent. In some embodiments, the present application provides a method for treating head and neck cancer that a second-line and higher-line treatment have failed to treat, comprising administering to a patient in need thereof the compound I or the pharmaceutically acceptable salt thereof, and the at least one second therapeutic agent. In one embodiment, the present application provides a method for treating refractory and recurrent head and neck cancer, comprising administering to a patient in need thereof the compound I or the pharmaceutically acceptable salt thereof, and at the least one second therapeutic agent. In some embodiments, the compound I or the pharmaceutically acceptable salt thereof and the at least one second therapeutic agent are used in combination for treating primary or secondary head and neck cancer. In some embodiments, the head and neck cancer is chemotherapy-intolerant head and neck cancer.

In some embodiments of the present application, the patient has not previously received systemic chemotherapy. In some embodiments, the patient has previously received surgical treatment, radiation therapy, induction chemotherapy, concurrent chemotherapy, and/or adjuvant chemotherapy. In some specific embodiments, the patient has not previously received systemic chemotherapy, but has received surgical treatment, radiation therapy, induction chemotherapy, concurrent chemotherapy, and/or adjuvant chemotherapy. In some specific embodiments, progressive disease recurs after the patient has achieved complete response following surgical treatment, radiation therapy, induction chemotherapy, concurrent chemotherapy and/or adjuvant chemotherapy. In some specific embodiments, the patient has failed to achieve complete response or partial response following surgical treatment, radiation therapy, induction chemotherapy, concurrent chemotherapy and/or adjuvant chemotherapy. In some specific embodiments, the cancer metastasizes after the patient has received surgical treatment, radiation therapy, induction chemotherapy, concurrent chemotherapy and/or adjuvant chemotherapy. The administration regimen can be determined according to a combination of factors such as the activity and toxicity of the drug, and tolerance of the subject. In some embodiments of the present application, for the use or method of treatment, the second therapeutic agent can be administered according to administration regimens including but not limited to, once, twice, three times or four times daily (qd), every other day (qod), every 3 days (q3d), every 4 days (q4d), every 5 days (q5d), every week (q1w), every 2 weeks (q2w), every 3 weeks (q3w) or every 4 weeks (q4w), for example, twice daily (bid), twice weekly (biw), three times daily (tid), four times daily (qid), or the like. In some embodiments of the present application, for the use or method of treatment, the second therapeutic agent can be administered according to an intermittent administration regimen. The intermittent administration regimen includes a

treatment period and an interruption period, for example, the second therapeutic agent is administered daily in the treatment period, and then interrupted in the interruption period, followed by entering the treatment period and then the interruption period. Such an intermittent administration can be repeated multiple times.

In some embodiments of the present application, for the use or method of treatment, the compound I or the pharmaceutically acceptable salt thereof can be administered according to administration regimens including but not limited to, once daily at a dose of 6 mg, 8 mg, 10 mg or 12 mg, consecutively 2-week treatment and then 1-week interruption; and/or, consecutively 2-week treatment and then 2-week interruption.

In some embodiments, the second therapeutic agent and the compound I or the pharmaceutically acceptable salt thereof each have the same or different treatment cycles. In some specific embodiments, the second therapeutic agent and the compound I or the pharmaceutically acceptable salt thereof have the same treatment cycle, e.g., a 1-week, 2-week, 3-week, or 4-week treatment cycle. In some specific embodiments, the second therapeutic agent and the compound I or the pharmaceutically acceptable salt thereof have a 3-week treatment cycle.

In some embodiments, the present application provides a combined pharmaceutical composition, which is a formulation suitable for administration in a single treatment cycle (e.g., a 3-week treatment cycle), comprising 84-168 mg, preferably 112-168mg, of the compound I or the pharmaceutically acceptable salt thereof and at least one second therapeutic agent. The compound I or the pharmaceutically acceptable salt thereof can be packaged separately in multiple aliquots (e.g., 2, 7, 14, 28 or more aliquots).

In addition, the present application provides a kit for use in treating head and neck cancer, comprising the compound I or the pharmaceutically acceptable salt thereof and the at least one second therapeutic agent each packaged separately, and optionally a package insert.

In some specific embodiments of the present application, the objective response rate of the compound I or the pharmaceutically acceptable salt thereof and the combined pharmaceutical composition comprising the compound I or the pharmaceutically acceptable salt thereof in clinical trials for human patients diagnosed with head and neck cancer is about 10% or more, preferably about 15% or more, further preferably about 20% or more, more preferably about 30% or more, and especially 35% or more; the disease control rate is 50% or more, preferably 60% or more, more preferably 70% or more, still more preferably 80% or more, and particularly 90% or more.

Head and neck cancer

In some embodiments of the present application, the head and neck cancer is head and neck squamous cell carcinoma. In some embodiments, the head and neck cancer is head and neck adenocarcinoma. In some embodiments, the head and neck cancer includes, but is not limited to, neck tumors, otorhinolaryngological tumors and oromaxillofacial tumors. In some embodiments, the head and neck cancer includes, but is not limited to, oral cancer, laryngeal cancer, parotid gland cancer, thyroid cancer, nasopharyngeal carcinoma, nasal cavity cancer, sinus cancer, paranasal sinus cancer, oropharyngeal cancer, floor of mouth cancer, hypopharyngeal cancer, palate cancer, gum cancer, tongue cancer, tongue mucosal squamous cell carcinoma, buccal mucosal carcinoma, lip cancer, salivary gland cancer, tonsil cancer, and the like.

Primary head and neck adenocarcinomas may occur in the salivary gland or nasal cavity and sinus region. Salivary gland cancer can originate from the major salivary glands (parotid gland, submandibular gland and sublingual gland) and also from the minor salivary glands which are distributed in the oral cavity and submucosa of the upper respiratory digestive tract, including the oral cavity (especially the palate), sinus, larynx and pharynx. Salivary gland cancer has low incidence rate and is not common clinically, with the incidence rate less than 5% of that of head and neck malignant tumors. The annual incidence rates reported worldwide vary from 0.3 to 4 new cases per 100,000 people, and experience with its treatment is based largely on retrospective case series studies. The most common salivary gland malignant tumors include mucoepidermoid carcinoma, adenoid cystic carcinoma and lymphatic epithelial carcinoma. Other head and neck adenocarcinomas further include pleomorphic adenocarcinomas (pleomorphic low-grade malignant adenocarcinomas), salivary duct carcinomas, myoepithelial carcinomas (epithelial tumors), non-specific adenocarcinomas, non-specific clear cell carcinomas, cystadenocarcinomas, sebaceous adenocarcinomas, sebaceous lymphoid adenocarcinomas, mucinous adenocarcinomas, and epithelial-myoepithelial carcinomas (epithelial tumors). Nasal cavity and sinus adenocarcinomas may resemble common salivary gland tumors, serous mucinous tumors, gut-type differentiated tumors and other rare variants.

In some embodiments, the head and neck cancer is occult cancer or unapparent primary head and neck cancer. In some embodiments, the clinical stage of the head and neck cancer includes, but is not limited to, locally advanced, and/or advanced (e.g., stage IIIB/IV) head and neck cancer. In some embodiments, the head and neck cancer is metastatic head and neck cancer. The metastatic head and neck cancer includes, but is not limited to, distant metastasis, single metastasis, disseminated metastasis and diffuse metastasis of a lesion; the metastatic lesions include, but are not limited to, lung, lymph node, pleura, bone, brain, pericardium, adrenal gland and liver. In some embodiments, the head and neck cancer is head and neck cancer with brain metastasis. In some embodiments, the head and neck cancer is head

and neck cancer with lymph node metastasis.

In some embodiments of the present application, the head and neck cancer is recurrent head and neck cancer; in certain embodiments, the head and neck cancer is refractory head and neck cancer; in certain embodiments, the head and neck cancer is unresectable head and neck cancer. In some embodiments, the head and neck cancer is one that chemotherapeutic agents and/or targeted agents have failed to treat. In some embodiments, the head and neck cancer is head and neck cancer that has been treated with at least two chemotherapeutic drugs. In some embodiments, the head and neck cancer is head and neck cancer that a second-line and higher-line chemotherapy have failed to treat. In one embodiment, the head and neck cancer is refractory and recurrent head and neck cancer, wherein the "refractory and recurrent head and neck cancer" refers to head and neck cancer that has failed to achieve response after chemotherapy, and head and neck cancer that has achieved response but progressed within 3 months after chemotherapy.

In some embodiments of the present application, the nasopharyngeal carcinoma includes, but is not limited to, keratinizing squamous cell carcinoma, non-keratinizing squamous cell carcinoma, and the latter may include a differentiated type and an undifferentiated type, such as undifferentiated non-keratinizing carcinoma in the roof and posterior wall of the nasopharynx.

In some embodiments, the nasopharyngeal carcinoma includes, but is not limited to, an orthotopic nasopharyngeal carcinoma and an invasive nasopharyngeal carcinoma. In some embodiments, the invasive nasopharyngeal carcinoma includes, but is not limited to, squamous cell carcinoma (including well, moderately, and poorly differentiated types), adenocarcinoma (including well, moderately, and poorly differentiated types), micro-invasive carcinoma, alveolar cell nuclear carcinoma, or undifferentiated nasopharyngeal carcinoma. In some embodiments, the adenocarcinoma further comprises papillary adenocarcinoma or columnar cell adenocarcinoma.

In some embodiments, the clinical stage of nasopharyngeal carcinoma includes, but is not limited to: stage I, stage II, stage III, stage IVa and stage IVb, for example, T1NOM0, T2N0-1M0, T0-2N1M0, T3N0-2M0, T0-3N1M0, T4N0-3M0, T0-4N3M0, or any T, any N, M1.

In some embodiments, the nasopharyngeal carcinoma is type I keratinizing squamous cell carcinoma. In some embodiments, the nasopharyngeal carcinoma is type II non-keratinizing squamous cell carcinoma. In some embodiments, the nasopharyngeal carcinoma is type II undifferentiated non-keratinizing squamous cell carcinoma. In some embodiments, the nasopharyngeal carcinoma is type II differentiated non-keratinizing squamous cell carcinoma.

In some embodiments, the nasopharyngeal carcinoma is metastatic nasopharyngeal carcinoma. In some embodiments, the nasopharyngeal carcinoma is recurrent and/or refractory and/or advanced nasopharyngeal carcinoma. In some embodiments, the nasopharyngeal carcinoma is unresectable nasopharyngeal carcinoma; in certain embodiments, the nasopharyngeal carcinoma is nasopharyngeal carcinoma that has been treated with at least two chemotherapeutic drugs.

In some embodiments, the chemotherapy includes first-line chemotherapy and second-line chemotherapy, including but not limited to one or more of taxanes (e.g., paclitaxel, docetaxel), fluorouracil, cisplatin and cyclophosphamide. It will be understood by those skilled in the art that a patient can also receive radiotherapy prior to, concurrently with, or subsequent to the chemotherapy.

Second therapeutic agent

The second therapeutic agent described herein includes, but is not limited to, a chemotherapeutic agent, a small molecule targeted anti-tumor agent, an immunotherapeutic agent, and a macromolecular antibody drug.

As used herein, the second therapeutic agents includes, but is not limited to, one or more of platinum agents, fluoropyrimidine derivatives, camptothecin analogs, taxanes, vinca alkaloids, anthracyclines, antibiotics, podophyllums, antimetabolites; and examples that can be listed include, but are not limited to: one or more of platinum agents (e.g., oxaliplatin, cisplatin, carboplatin, miriplatin, nedaplatin, dicycloplatin, lobaplatin, triplatin tetranitrate, phenanthriplatin, picoplatin, satraplatin), fluoropyrimidine derivatives (e.g., gemcitabine, capecitabine, ancitabine, fluorouracil (5-fluorouracil), difuradin, doxifluridine, tegafur, carmofur, trifluridine), taxanes (e.g., paclitaxel, albumin-bound paclitaxel, and docetaxel), camptothecin analogs (e.g., camptothecin, hydroxycamptothecin, 9-aminocamptothecin, 7-ethylcamptothecin, irinotecan, topotecan), vinca alkaloids (vinorelbine, vinblastine, vincristine, vindesine, vinflunine, , anthracyclines (epirubicin, adriamycin, daunorubicin, pirarubicin, amrubicin, idarubicin, mitoxantrone, aclarubicin, valrubicin, zorubicin, pixantrone, ), cytarabine, thioguanine, pemetrexed, carmustine, melphalan, etoposide, teniposide, mitomycin, ifosfamide, cyclophosphamide, azacitidine, methotrexate, bendamustine, liposomal doxorubicin, actinomycin D (dactinomycin), bleomycin, pingyangmycin, temozolomide, dacarbazine, peplomycin, eribulin, plinabulin, sapacitabine, treosulfan, 153Sm-EDTMP, tegafur-gimeracil-oteracil potassium, and encequidar.

In certain specific embodiments, the second therapeutic agent is one or more of platinum agents including but not limited to, cisplatin, carboplatin, nedaplatin, oxaliplatin, miriplatin, triplatin tetranitrate, phenanthriplatin, picoplatin, satraplatin, lobaplatin, and the like.

If desired, the second therapeutic agent is used in combination with a chemotherapeutic adjuvant including but

not limited to, calcium folinate (CF), leucovorin, mesna, bisphosphonate, amifostine, and hematopoietic cell colony stimulating factor (CSF). In some embodiments, the chemotherapeutic adjuvant is calcium folinate (CF), mesna, and leucovorin.

In some embodiments, the second therapeutic agent is an immunotherapeutic agent including but not limited to one or more of interferon (interferon $\alpha$, interferon $\alpha$-1b, interferon $\alpha$-2b), interleukin, sirolimus, everolimus, ridaforolimus, and temsirolimus.

In some embodiments, the second therapeutic agent is a small molecule targeted anti-tumor agent including but not limited to protein kinase inhibitors. The protein kinase inhibitors include, but are not limited to tyrosine kinase inhibitors, serine and/or threonine kinase inhibitors, and poly ADP-ribose polymerase (PARP) inhibitors. The targets for the inhibitors include, but are not limited to, Fascin-1 protein, HDAC (histone deacetylase), proteasome, CD38, SLAMF7 (CS1/CD319/CRACC), RANKL, epidermal growth factor receptor (EGFR), anaplastic lymphoma kinase (ALK), MET gene, ROS1 gene, HER2 gene, RET gene, BRAF gene, PI3K signaling pathway, discoidin death receptor 2 (DDR2) gene, fibroblast growth factor receptor 1 (FGFR1), neurotrophic tyrosine kinase type 1 receptor (NTRK1) gene, and KRAS gene. The targets for the small molecule targeted anti-tumor agents also include cyclooxygenase-2 (COX-2), apurinic/apyrimidinic endonuclease-1 (APE1), vascular endothelial growth factor receptor (VEGFR), chemokine receptor-4 (CXCR-4), matrix metalloproteinase (MMP), insulin-like growth factor receptor (IGF-1R), Ezrin, pigment epithelium derived factor (PEDF), AS, ES, osteoprotegerin (OPG), Src, IFN, activated leukocyte cell adhesion molecule (ALCAM), HSP,JIP1, GSK-3$\beta$ (glycogen synthase kinase-3$\beta$), cell cycle regulatory protein (CyclinD1), cyclin-dependent kinase (CDK4), tissue metalloproteinase inhibitor (TIMP1), THBS3, parathyroid hormone-related protein receptor 1 (PTHR1), tumor endothelial marker 7 (TEM7), COPS3, and cathepsin K. Examples of small molecule targeted anti-tumor agents include, but are not limited to, one or more of imatinib, sunitinib, nilotinib, bosutinib, saracatinib, pazopanib, trabectedin, regorafenib, cediranib, bortezomib, panobinostat, carfilzomib, ixazomib, apatinib, erlotinib, afatinib, crizotinib, ceritinib, vemurafenib, dabrafenib, cabozantinib, gefitinib, dacomitinib, osimertinib, olmutinib, alectinib, brigatinib, lorlatinib, trametinib, larotrectinib, icotinib, lapatinib, vandetanib, selumetinib, sorafenib, olmutinib, savolitinib, fruquintinib, entrectinib, dasatinib, ensartinib, lenvatinib, itacitinib, pyrotinib, binimetinib, erdafitinib, axitinib, neratinib, cobimetinib, acalabrutinib, famitinib, masitinib, ibrutinib, rociletinib, nintedanib, lenalidomide, LOXO-292, vorolanib, bemcentinib, capmatinib, entrectinib, TAK-931, ALT-803, palbociclib, famitinib L-malate, LTT-462, BLU-667, ningetinib, tipifarnib, poziotinib, DS-1205c, capivasertib, SH-1028, DMBG, seliciclib, OSE-2101, APL-101, berzosertib, idelalisib, lerociclib, ceralasertib, PLB-1003, tomivosertib, AST-2818, SKLB-1028, D-0316, LY-3023414, allitinib, MRTX-849, AP-32788, AZD-4205, lifirafenib, vactosertib, mivebresib, napabucasin, sitravatinib, TAS-114, molibresib, CC-223, rivoceranib, CK-101, LXH-254, simotinib, GSK-3368715, TAS-0728, masitinib, tepotinib, HS-10296, AZD-4547, merestinib, olaptesed pegol, galunisertib, ASN-003, gedatolisib, defactinib, lazertinib, CKI-27, S-49076, BPI-9016M, RF-A-089, RMC-4630, AZD-3759, antroquinonol, SAF-189s, AT-101, TTI-101, naputinib, LNP-3794, HH-SCC-244, ASK-120067, CT-707, epitinib succinate, tesevatinib, SPH-1188-11, BPI-15000, copanlisib, niraparib, olaparib, veliparib, talazoparib tosylate, DV-281, siremadlin, telaglenastat, MP-0250, GLG-801, ABTL-0812, bortezomib, tucidinostat, vorinostat, resminostat, epacadostat, tazemetostat, entinostat, mocetinostat, quisinostat, LCL-161, and KML-001. In some embodiments, the small molecule targeted anti-tumor agent is one or more of sorafenib, erlotinib, afatinib, crizotinib, ceritinib, vemurafenib, dabrafenib, cabozantinib, gefitinib, dacomitinib, osimertinib, alectinib, brigatinib, lorlatinib, trametinib, larotrectinib, icotinib, lapatinib, vandetanib, selumetinib, olmutinib, savolitinib, fruquintinib, entrectinib, dasatinib, ensartinib, lenvatinib, itacitinib, pyrotinib, binimetinib, erdafitinib, axitinib, neratinib, cobimetinib, acalabrutinib, famitinib, masitinib, ibrutinib, and nintedanib.

In some embodiments, the second therapeutic agent is a macromolecular antibody drug. The targets for the macromolecular antibody drug include, but are not limited to, any one or more of PD-1, PD-L1, cytotoxic T-lymphocyte antigen 4 (CTLA-4), platelet-derived growth factor receptor $\alpha$ (PDGFR-$\alpha$), vascular endothelial growth factor (VEGF), human epidermal growth factor receptor-2 (HER2), epidermal growth factor receptor (EGFR), ganglioside GD2, B cell surface protein CD20, B cell surface protein CD52, B cell surface protein CD38, B cell surface protein CD319, B cell surface protein CD30 and B cell surface protein CD19/CD3.

In some embodiments, the antibody drug is an inhibitor for the interaction between the PD-1 receptor and its ligand PD-L1; in some embodiments, the antibody drug is a cytotoxic T-lymphocyte antigen 4 (CTLA-4) inhibitor. In some embodiments, the antibody drug is a platelet-derived growth factor receptor $\alpha$ (PDGFR-$\alpha$) inhibitor. In some embodiments, the antibody drug is an epidermal growth factor receptor (EGFR) inhibitor.

In some embodiments, the inhibitor for the interaction between a PD-1 receptor and its ligand PD-L1 is an antibody or an antigen-binding portion thereof that binds to programmed death receptor 1 (PD-1) and/or inhibits PD-1 activity, or an antibody or an antigen-binding portion thereof that binds to programmed death ligand 1 (PD-L1) and/or inhibits PD-L1 activity, e.g., an anti-PD-1 antibody or an anti-PD-L1 antibody. In some specific embodiments, the antibody or the antigen-binding portion thereof is (a) an anti-PD-1 monoclonal antibody or an antigen-binding fragment thereof that specifically binds to human PD-1 and blocks the binding of human PD-L1 to human PD-1; or (b) an anti-PD-L1 monoclonal antibody or an antigen-binding fragment thereof that specifically binds to human PD-L1 and blocks the binding of human PD-L1 to human PD-1.

In some embodiments, the anti-PD-1 or anti-PD-L1 antibody is an anti-PD-1 or anti-PD-L1 monoclonal antibody. In some embodiments, the anti-PD-1 or anti-PD-L1 antibody is a human or murine antibody.

In some embodiments, the anti-PD-1 antibody can be selected from the group consisting of any one or more of nivolumab, pembrolizumab, durvalumab, toripalimab (JS-001), sintilimab (IBI308), camrelizumab, tislelizumab (BGB-A317), genolimzumab (GB226), lizumab (LZM009), HLX-10, BAT-1306, AK103 (HX008), AK104 (Akesobio), CS1003, SCT-I10A, F520, SG001 and GLS-010.

In some embodiments, the anti-PD-L1 antibody can be selected from the group consisting of any one or more of atezolizumab, avelumab, durvalumab, KL-A167, SHR-1316, BGB-333, JS003, STI-A1014 (ZKAB0011), KN035, MSB2311, HLX-20 and CS-1001.

In some specific embodiments, the anti-PD-1 antibody is toripalimab.

In some specific embodiments, the anti-PD-1 antibody is pembrolizumab.

In some embodiments, the inhibitor for cytotoxic T-lymphocyte antigen 4 (CTLA-4) is an anti-CTLA-4 antibody. In some specific embodiments, the anti-CTLA-4 antibody is an anti-CTLA-4 monoclonal antibody.

In some embodiments, the anti-CTLA-4 antibody can be selected from the group consisting of any one or more of ipilimumab, tremelimumab, AGEN-1884, BMS-986249, BMS-986218, AK-104 and IBI310.

In some specific embodiments, the anti-CTLA-4 antibody is ipilimumab.

In some embodiments, the platelet-derived growth factor receptor $\alpha$ (PDGFR-$\alpha$) inhibitor is an anti-PDGFRa antibody. In some specific embodiments, the anti-PDGFRa antibody is an anti-PDGFRa monoclonal antibody.

In some specific embodiments, the anti-PDGFRa antibody is olaratumab.

In some embodiments, the antibody drug is an epidermal growth factor receptor (EGFR) inhibitor or an anti-EGFR antibody. In some specific embodiments, the anti-EGFR antibody is an anti-EGFR monoclonal antibody. In some specific embodiments, the anti-EGFR antibody is cotuximab.

In some specific embodiments, the antibody drug can further include, but is not limited to, any one or more of bevacizumab, ramucirumab, pertuzumab, trastuzmab, cotuximab, nimotuzumab, panitumumab, necitumumab, dinutuximab, rituximab, ibritumomab, ofatumumab, obinutuzumab, alemtuzumab, daratumumab, gemtuzumab, elotuzumab, brentuximab, inotuzumab ozogamicin, and blinatumomab.

In some embodiments, the second therapeutic agent is one or more of methotrexate, cisplatin, carboplatin, paclitaxel, docetaxel fluorouracil, vinorelbine, bleomycin, ifosfamide, mesna, leucovorin, mitoxantrone, pirarubicin, daunorubicin, cytarabine, thioguanine, etoposide, harringtonine, gemcitabine, cotuximab, and epirubicin.

In some embodiments, the second therapeutic agent is methotrexate.

In some specific embodiments, the second therapeutic agent is methotrexate (40 mg/m$^2$ IV on day 1 once every 7 days).

In some embodiments, the second therapeutic agent is paclitaxel.

In some specific embodiments, the second therapeutic agent is paclitaxel (250 mg/m$^2$ CIV on day 1, repeated every 21 days).

In some embodiments, the second therapeutic agent is carboplatin and 5-fluorouracil.

In some embodiments, the second therapeutic agent is cisplatin and cotuximab.

In some embodiments, the second therapeutic agent is carboplatin and cotuximab.

In some embodiments, the second therapeutic agent is gemcitabine and vinorelbine.

In some embodiments, the second therapeutic agent is gemcitabine and paclitaxel.

In some embodiments, the second therapeutic agent is a DF regimen, specifically cisplatin and fluorouracil.

In some embodiments, the second therapeutic agent is a PC regimen, specifically paclitaxel and carboplatin.

In some embodiments, the second therapeutic agent is a DA regimen, specifically daunorubicin and cytarabine.

In some embodiments, the second therapeutic agent is an ME regimen, specifically mitoxantrone and etoposide.

In some embodiments, the second therapeutic agent is a GP regimen, specifically gemcitabine and cisplatin.

In some embodiments, the second therapeutic agent is an FP regimen, specifically 5-fluorouracil and cisplatin.

In some embodiments, the second therapeutic agent is an AP regimen, specifically adriamycin and cisplatin.

In some embodiments, the second therapeutic agent is an IE regimen, specifically ifosfamide, mesna and etoposide.

In some embodiments, the second therapeutic agent is docetaxel, cisplatin and 5-fluorouracil.

In some embodiments, the second therapeutic agent is cisplatin, epirubicin and paclitaxel.

In some embodiments, the second therapeutic agent is cisplatin, 5-fluorouracil and cotuximab.

In some embodiments, the second therapeutic agent is carboplatin, 5-fluorouracil and cotuximab.

In some embodiments, the second therapeutic agent is cisplatin, docetaxel and paclitaxel.

In some embodiments, the second therapeutic agent is carboplatin, docetaxel and paclitaxel.

In some embodiments, the second therapeutic agent is carboplatin, paclitaxel and gemcitabine.

In some embodiments, the second therapeutic agent is an NMB regimen, specifically vinorelbine, methotrexate and bleomycin.

In some embodiments, the second therapeutic agent is a PIC regimen, specifically paclitaxel, ifosfamide, mesna and cisplatin.

In some embodiments, the second therapeutic agent is a DLF regimen, specifically cisplatin, fluorouracil and leucovorin.

In some embodiments, the second therapeutic agent is a PBF regimen, specifically cisplatin, bleomycin and fluorouracil.

In some embodiments, the second therapeutic agent is an MFC regimen, specifically mitoxantrone, fluorouracil and carboplatin.

In some embodiments, the second therapeutic agent is a TPF regimen, specifically pirarubicin, cisplatin and fluorouracil.

In some embodiments, the second therapeutic agent is a DAT regimen, specifically daunorubicin, cytarabine, thioguanine and etoposide.

In some embodiments, the second therapeutic agent is an HA regimen, specifically harringtonine, cytarabine and thioguanine.

In some embodiments, the second therapeutic agent is an HOAP regimen, specifically harringtonine, vincristine, cytarabine and prednisone.

In certain specific embodiments, the second therapeutic agent is tegafur-gimeracil-oteracil potassium.

In certain embodiments, the second therapeutic agent is sintilimab.

In certain embodiments, the second therapeutic agent is capecitabine.

Compound I or pharmaceutically acceptable salt thereof

The chemical name of the compound I is 1-[[[4-(4-fluoro-2-methyl-1*H*-indol-5-yl)oxy-6-methoxyquinolin-7-yl]oxy]methyl]cyclopropylamine, which has the following structural formula:

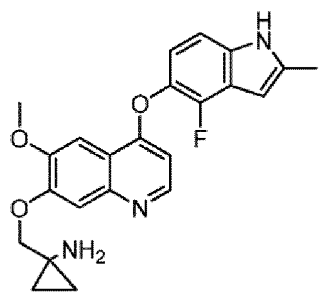

compound I.

In the present application, anlotinib refers to compound I in any case.

The compound I can be administered in its free base form, or in the form of its salt, hydrate, or its prodrug that may convert *in vivo* into the free base form. For example, within the scope of the present application, the pharmaceutically acceptable salt of the compound I can be generated from various organic and inorganic acids according to methods well known in the art.

In some embodiments, the compound I or the pharmaceutically acceptable salt thereof is administered in the form of a hydrochloride salt. In some embodiments, the compound I or the pharmaceutically acceptable salt thereof is administered in the form of a monohydrochloride salt. In some embodiments, the compound I or the pharmaceutically acceptable salt thereof is administered in the form of a dihydrochloride salt. In some embodiments, the compound I or the pharmaceutically acceptable salt thereof is administered in the form of a crystalline hydrochloride salt. In a specific embodiment, the compound I or the pharmaceutically acceptable salt thereof is administered in the form of a crystalline dihydrochloride salt.

The compound I or the pharmaceutically acceptable salt thereof and the second therapeutic agent can be administered via multiple routes of administration, including but not limited to routes selected from the group consisting of oral, parenteral, intraperitoneal, intravenous, intra-arterial, transdermal, sublingual, intramuscular, rectal, transbuccal, intranasal, inhalational, vaginal, intraocular, topical, subcutaneous, intralipid, intra-articular and intrathecal routes. In a specific embodiment, the compound I or the pharmaceutically acceptable salt thereof and the second therapeutic agent is administered orally.

The amount of the compound I or the pharmaceutically acceptable salt thereof and the second therapeutic agent administered can be determined according to the severity of the disease, the response of the disease, any treatment-related toxicity, and the age and health of a subject. In some embodiments, the compound I or the pharmaceutically acceptable salt thereof is administered at a daily dose of 3 mg to 30 mg. In some embodiments, the compound

I or the pharmaceutically acceptable salt thereof is administered at a daily dose of 5 mg to 20 mg. In some embodiments, the compound I or the pharmaceutically acceptable salt thereof is administered at a daily dose of 8 mg to 16 mg. In some embodiments, the compound I or the pharmaceutically acceptable salt thereof is administered at a daily dose of 10 mg to 14 mg. In a specific embodiment, the compound I or the pharmaceutically acceptable salt thereof is administered at a daily dose of 8 mg. In a specific embodiment, the compound I or the pharmaceutically acceptable salt thereof is administered at a daily dose of 10 mg. In a specific embodiment, the compound I or the pharmaceutically acceptable salt thereof is administered at a daily dose of 12 mg. In the present application, for example, for tablets or capsules, "comprising 12 mg of the compound I or the pharmaceutically acceptable salt thereof on a unit dose basis" means that the final tablets or capsules each comprise 12 mg of the compound I.

The compound I or the pharmaceutically acceptable salt thereof and the second therapeutic agent can be administered once or multiple times daily. In some embodiments, the compound I or the pharmaceutically acceptable salt thereof is administered once daily. In one embodiment, the compound I or the pharmaceutically acceptable salt thereof is administered once daily in the form of a solid oral formulation.

In the above methods of treatment, the administration regimen can be determined according to a combination of factors such as the activity and toxicity of the drug and tolerance of the subject. Preferably, the compound I or the pharmaceutically acceptable salt thereof is administered according to an intermittent administration regimen. The intermittent administration regimen includes a treatment period and an interruption period. The compound I or the pharmaceutically acceptable salt thereof can be administered once or multiple times daily in the treatment period. For example, the compound I or the pharmaceutically acceptable salt thereof is administered daily in the treatment period, and then interrupted in the interruption period, followed by entering the treatment period and then the interruption period. Such an intermittent administration can be repeated multiple times. The ratio of the treatment period to the interruption period in days is 2:0.5 to 2:5, preferably 2:0.5 to 2:3, more preferably 2:0.5 to 2:2, and even more preferably 2:0.5 to 2:1.

In some embodiments, the compound I or the pharmaceutically acceptable salt thereof is administered according to the administration regimen of consecutive 2-week treatment and then 2-week interruption. In some embodiments, the treatment is administered once daily for 14 consecutive days and then interrupted for 14 days; and then administered once daily for 14 consecutive days and then interrupted for 14 days, etc. Such an intermittent regimen in consecutively 2-week treatment and then 2-week interruption can be repeated multiple times.

In some embodiments, the compound I or the pharmaceutically acceptable salt thereof is administered according to the administration regimen of consecutive 2-week treatment and then 1-week interruption. In some embodiments, the compound I or the pharmaceutically acceptable salt thereof is administered according to the administration regimen: once daily, consecutive 14-day treatment and then 7-day interruption; followed by once daily, consecutive 14-day treatment and then 7-day interruption. Such an intermittent administration regimen of consecutive 2-week treatment and then 1-week interruption can be repeated multiple times. In a specific embodiment, the compound I or the pharmaceutically acceptable salt thereof is administered on days 1-14 of each 21-day treatment cycle.

In some embodiments, the compound I or the pharmaceutically acceptable salt thereof is administered according to the administration regimen of consecutive 5-day treatment and then 2-day interruption. In some embodiments, the compound I or the pharmaceutically acceptable salt thereof is administered according to the administration regimen: once daily, consecutive 5-day treatment and then 2-day interruption; followed by once daily, consecutively 5-day treatment and then 2-day interruption. Such an intermittent administration regimen of consecutively 5-day treatment and then 2-day interruption can be repeated multiple times.

In certain specific embodiments, the compound I or the pharmaceutically acceptable salt thereof is administered according to the intermittent administration regimen: once daily at a dose of 12 mg, 2-week treatment and then 1-week interruption.

## Pharmaceutical combination

In certain embodiments, the compound I or the pharmaceutically acceptable salt thereof is used in combination with surgical resection and/or radiation therapy.

The components of the pharmaceutical composition described herein can be used optionally in combination with one or more pharmaceutically acceptable carriers, wherein the components can independently, or some or all of the components can together comprise a pharmaceutically acceptable carrier and/or excipient. The pharmaceutical composition described herein can be formulated separately, or some or all of the pharmaceutical combinations can be co-formulated. Preferably, the components of the pharmaceutical composition are formulated separately or each formulated into a suitable pharmaceutical composition. In some embodiments, the pharmaceutical composition of the present application can be formulated into a pharmaceutical composition which is suitable for a single dose or multiple doses. In some specific embodiments, the pharmaceutical composition comprising the compound I or the pharmaceutically acceptable salt thereof may be selected from the group consisting of solid pharmaceutical compositions including but not limited to, tablets and capsules.

The components of the pharmaceutical composition of the present application can be administered separately, or some or all of the components are co-administered. The components of the pharmaceutical composition of the present application can be administered in a substantially asynchronous manner, or some or all of the components are administered in a substantially synchronous manner.

The components in the pharmaceutical composition of the present application can be administered independently, or some or all of the components are co-administered in proper routes including, but not limited to, oral administration or parenteral administration (by intravenous, intramuscular, topical or subcutaneous routes). In some embodiments, the components of the pharmaceutical composition of the present application can be administered independently, or some or all of the components are co-administered via oral administration or injection, for example, intravenous injection or intraperitoneal injection.

The components in the pharmaceutical composition of the present application can be formulated independently in suitable dosage forms, or some or all of the components are co-formulated in a suitable dosage form including, but not limited to, tablet, lozenge, pill, capsule (for example, hard capsule, soft capsule, enteric capsule and microcapsule), elixir, granule, syrup, injection (intramuscular, intravenous and intraperitoneal), granule, emulsion, suspension, solution, dispersant and dosage forms of sustained-released preparations for oral or non-oral administration.

In some embodiments of the present application, the pharmaceutical composition is a fixed combination. In some embodiments, the fixed combination is in the form of a solid pharmaceutical composition or a liquid pharmaceutical composition.

In some embodiments of the present application, the pharmaceutical composition is a non-fixed combination. In some embodiments, the second therapeutic agent and the compound I or the pharmaceutically acceptable salt thereof in the non-fixed combination are each in the form of a pharmaceutical composition.

In some embodiments of the present application, the compound I or the pharmaceutically acceptable salt thereof is administered concurrently or sequentially with one or more second therapeutic agents. In certain embodiments, the one or more second therapeutic agents have been administered to the subject prior to administration of, or combination with, the compound I or the pharmaceutically acceptable salt thereof. In certain embodiments, the one or more second therapeutic agents are administered to the subject after administration of, or combination with, the compound I or the pharmaceutically acceptable salt thereof. In certain embodiments, the compound I or the pharmaceutically acceptable salt thereof has been administered to the subject prior to administration of, or combination with, the one or more second therapeutic agents. In certain embodiments, the compound I or the pharmaceutically acceptable salt thereof is administered to the subject after administration of, or combination with, the one or more second therapeutic agents. In some embodiments, when the compound I or the pharmaceutically acceptable salt thereof is administered to a subject in combination with one or more second therapeutic agents, the compound I or the pharmaceutically acceptable salt thereof and the one or more second therapeutic agents are administered to the subject sequentially. In certain embodiments, one or more second therapeutic drugs are not effective in treating cancer. In some embodiments, the second therapeutic drug is any anti-cancer agent described herein or known in the art.

In some embodiments, also provided is a kit of a pharmaceutical composition for use in treating head and neck cancer, comprising: (a) a first pharmaceutical composition comprising a chemotherapeutic agent as an active ingredient; and (b) a second pharmaceutical composition comprising the compound I or the pharmaceutically acceptable salt thereof as an active ingredient. In some embodiments, also provided is a kit of a pharmaceutical composition for use in treating head and neck cancer, comprising: (a) a first pharmaceutical composition comprising a small molecule targeted anti-tumor agent as an active ingredient; and (b) a second pharmaceutical composition comprising the compound I or the pharmaceutically acceptable salt thereof as an active ingredient. In some embodiments, also provided is a kit of a pharmaceutical composition for use in treating head and neck cancer, comprising: (a) a first pharmaceutical composition comprising an immunotherapeutic agent as an active ingredient; and (b) a second pharmaceutical composition comprising the compound I or the pharmaceutically acceptable salt thereof as an active ingredient. In some embodiments, also provided is a kit of a pharmaceutical composition for use in treating head and neck cancer, comprising: (a) a first pharmaceutical composition comprising a macromolecular antibody drug as an active ingredient; and (b) a second pharmaceutical composition comprising the compound I or the pharmaceutically acceptable salt thereof as an active ingredient.

In some embodiments, also provided is a kit of a pharmaceutical composition for use in treating head and neck cancer, comprising: (a) a first pharmaceutical composition comprising cotuximab and a platinum agent as active ingredients; and (b) a second pharmaceutical composition comprising the compound I or the pharmaceutically acceptable salt thereof as an active ingredient. In some embodiments, also provided is a kit of a pharmaceutical composition for use in treating head and neck cancer, comprising: (a) a first pharmaceutical composition comprising gemcitabine and a platinum agent as active ingredients; and (b) a second pharmaceutical composition comprising the compound I or the pharmaceutically acceptable salt thereof as an active ingredient. In some embodiments, also provided is a kit of a pharmaceutical composition for use in treating head and neck cancer, comprising: (a) a first pharmaceutical composition comprising gemcitabine and cisplatin as active ingredients; and (b) a second pharmaceutical composition comprising

the compound I or the pharmaceutically acceptable salt thereof as an active ingredient. In some embodiments, also provided is a kit of a pharmaceutical composition for use in treating head and neck cancer, comprising: (a) a first pharmaceutical composition comprising gemcitabine and paclitaxel as active ingredients; and (b) a second pharmaceutical composition comprising the compound I or the pharmaceutically acceptable salt thereof as an active ingredient.

Definitions and Description

As used herein, unless otherwise stated, the following terms used in the specification and claims shall have the following meanings for the purposes of the present invention.

As used herein, the term "treat", "treating" or "treatment" generally refers to obtaining a desired pharmacological and/or physiological effect. In terms of partially or fully stabilizing or curing the disease and/or a side effect of the disease, the effect can be therapeutic. As used herein, "treat", "treating" and "treatment" encompass any treatment to a disease in a subject, including (a) inhibiting a symptom of a disease, i.e., blocking the progression of the disease; or (b) alleviating a symptom of a disease, i.e., causing the remission of the disease or the symptom.

As used herein, the term "treatment failure" or "failure to treat" refers to intolerance of toxic and side effects, progressive disease during the treatment, or recurrence after the treatment, in particular means that patients who have received surgical treatment, radiotherapy, chemotherapy or multimodality therapy have disease progression or distant metastasis, and no other effective standard treatment means is available.

"Advanced stage" refers to a clinical stage of metastatic adenocarcinoma or a locally advanced stage at which local radical surgery or radiotherapy is useless.

As used herein, the term "subject" refers to a mammal, such as a rodent, feline, canine, and primate. Preferably, the subject according to the present application is a human. The term "patient" is a human. "Administer", "administering" or "administration" refers to physically introducing the composition comprising the therapeutic agent to the patient using any of a variety of methods and delivery systems known to those skilled in the art. Routes of administration include intravenous, intramuscular, subcutaneous, intraperitoneal, spinal or other parenteral routes, for example by injection or infusion. As used herein, the phrase "parenteral administration" refers to modes of administration apart from enteral and local administration, typically by injection, including but not limited to, intravenous, intramuscular, intra-arterial, intrathecal, intralymphatic, intralesional, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion and *in vivo* electroporation. In certain embodiments, the drug is administered via a non-parenteral route, and in certain embodiments, via oral administration. Other non-parenteral routes include local, epidermal or mucosal routes, for example, intranasal, vaginal, rectal, sublingual or local routes. Administration may also be performed, e.g., once, multiple times, and/or over one or more extended periods of time.

As an example, an "anti-cancer drug" promotes cancer regression in a patient or prevents further tumor growth. In certain embodiments, the drug promotes cancer regression to the point of eliminating the cancer. "Promoting cancer regression" means that the administration of a drug, alone or in combination with an anti-tumor agent results in a reduction of tumor growth or size, necrosis of the tumor, reduction in the severity of at least one disease symptom, increase in the frequency and duration of disease symptom-free stage. Furthermore, the terms "effective" and "effectiveness" with respect to treatment include pharmacological effectiveness and physiological safety. Pharmacological effectiveness refers to the ability of a drug to promote cancer regression in a patient. Physiological safety refers to the level of toxicity or other adverse physiological effects (adverse effects) at the cell, organ and/or organism level resulting from drug administration.

As an example for treating a tumor, an anti-cancer drug can inhibit cell growth or tumor growth by at least about 10%, at least about 20%, at least about 40%, at least about 60%, or at least about 80% relative to an untreated patient, or, in certain embodiments, relative to a subject treated with standard of care therapy. In other embodiments of the present application, tumor regression may be observed for a period of at least about 20 days, at least about 40 days, or at least about 60 days. Despite these final measurements of therapeutic effectiveness, the evaluation of drugs must also take into account "immune-related" response patterns.

An "immune-related" response pattern refers to the clinical response pattern often observed in cancer subjects treated with an immunotherapeutic agent that produces an anti-tumor effect by inducing a cancer-specific immune response or by altering the innate immune process. This response pattern is characterized by beneficial therapeutic effects following an initial increase in tumor burden or the appearance of new lesions, which would be classified as progressive disease and would be synonymous with drug failure in the evaluation of traditional chemotherapeutic agents. Thus, proper evaluation of immunotherapeutic agents may require long-term monitoring of the effect of these agents on target disease.

As used herein, the term "antibody" refers to a binding protein having at least one antigen-binding domain. The antibody and the fragment thereof of the present application can be an intact antibody or any fragment thereof. Thus, the antibody and the fragment thereof of the present application include a monoclonal antibody or a fragment thereof

and an antibody variant or a fragment thereof, as well as an immunoconjugate. Examples of the antibody fragment include a Fab fragment, a Fab' fragment, an $F(ab')_2$ fragment, an Fv fragment, an isolated CDR region, a single chain Fv molecule (scFv), an Fd fragment and other antibody fragments known in the art. The antibody and the fragment thereof may also include a recombinant polypeptide, a fusion protein, and a bispecific antibody. The anti-PD-L1 antibody and the fragment thereof disclosed herein can be of IgG1, IgG2, IgG3, or IgG4 isotype.

The term "isotype" refers to the class of antibodies encoded by the heavy chain constant region gene. In one embodiment, the anti-PD-1/anti-PD-L1 antibody and the fragment thereof disclosed herein are of the IgG1 or IgG4 isotype. The anti-PD-1/anti-PD-L1 antibody and the fragment thereof of the present application can be derived from any species, including but not limited to mouse, rat, rabbit, primate, llama, and human. The PD-1/PD-L1 antibody and the fragment thereof may be a chimeric antibody, a humanized antibody or an intact human antibody.

The term "humanized" means that the antigen-binding site in the antibody is derived from a non-human species and the variable region framework is derived from human immunoglobulin sequences. The humanized antibody may comprise substitutions in the framework regions such that the framework may not be an exact copy of the expressed human immunoglobulin or germline gene sequence.

The "isolated antibody" refers to an antibody that is substantially free of other antibodies having different antigenic specificities (e.g., an isolated antibody that specifically binds to PD-1/PD-L1 is substantially free of antibodies that specifically bind to antigens apart from PD-1/PD-L1). However, an isolated antibody that specifically binds to PD-1/PD-L1 may have cross-reactivity with other antigens (such as PD-1/PD-L1 molecules from different species). Furthermore, the isolated antibody may be substantially free of other cellular materials and/or chemicals.

The term "monoclonal antibody" ("mAb") refers to an antibody molecule of a single molecule composition. A monoclonal antibody composition exhibits a single binding specificity and affinity for a particular epitope or, in the case of bispecific monoclonal antibody, a dual binding specificity for two different epitopes. mAb is an example of the isolated antibody. mAbs can be produced by hybridoma techniques, recombinant techniques, transgenic techniques, or other techniques known to those skilled in the art. Examples of isolated monoclonal antibodies include, but are not limited to, nivolumab (Opdivo®), pembrolizumab (Keytruda®), durvalumab, avelumab, toripalimab (JS-001, Junshi Biosciences), sintilimab (IBI308, Innovent Biologics), camrelizumab (SHR-1210, Hengrui Medicine, refer to CN105026428B or WO2015085847A1), tislelizumab (BGB-A317, BeiGene), genolimzumab (GB226, Genor Biopharma), lizumab (LZM009, Livzon), HLX-10 (Henlius), BAT-1306 (Bio-Thera), HX008 (AK103, Akeso Bioscience/Hanzhong Pharmaceuticals), AK104 (Akeso Bioscience), CS1003 (CStone Pharmaceuticals), SCT-I10A (SinoCellTech), F520 (Shandong New Time Pharmaceutical/Lunan Pharmaceutical Group), SG001 (Sumgen Bio), GLS-010 (Goloria Pharceuticals), atezolizumab (Tecentriq®, Roche), avelumab (Bavencio®, Merck/Pfizer), durvalumab (Imfinzi®, AstraZeneca), KL-A167 (Kelun Pharmaceutical), SHR-1316 (Hengrui Medicine), BGB-333 (BeiGene), JS003 (Junshi Biosciences), STI-A1014 (ZKAB0011, Zhaoke Pharmaceutical), KN035 (Alphamab Oncology/3D Medicines ), MSB2311 (Mabspace Biosciences), HLX-20 (Henlius), CS-1001 (CStone Pharmaceuticals), etc.

An "antigen-binding portion" (also referred to as an "antigen-binding fragment") of an antibody refers to one or more fragments of the antibody that retain the ability to specifically bind to an antigen to which an intact antibody binds.

"Programmed death receptor-1 (PD-1)" refers to an immunosuppressive receptor belonging to the CD28 family. PD-1 is expressed primarily on previously activated T cells *in vivo* and binds to two ligands PD-L1 and PD-L2. As used herein, the term "PD-1" includes human PD-1 (hPD-1), variants, homologs and species homologs of hPD-1, and analogs having at least one common epitope with hPD-1.

"Programmed death ligand-1 (PD-L1)" is one of two cell surface glycoprotein ligands for PD-1 (the other is PD-L2), which down-regulates T cell activation and cytokine secretion upon binding to PD-1.

A "recurrent" cancer is one that regenerates at the initial site or a distant site after being responsive to initial treatment (e.g., surgery). A "locally recurrent" cancer is one that occurs, after treatment, at the same location as the previously treated cancer.

An "unresectable" cancer is one that cannot be removed by surgery.

A "metastatic" cancer refers to one that spreads from one part of the body (e.g., the head and neck) to another part of the body.

The use of alternatives (e.g., "or") shall be understood to refer to any one, two, or any combination of the alternatives. As used herein, the indefinite article "a" or "an" shall be understood to mean "one or more" of any listed or enumerated components.

The term "pharmaceutically acceptable" is used herein for those compounds, materials, compositions, and/or dosage forms which are, within the scope of sound medical judgment, suitable for use in contact with the tissues of human beings and animals without excessive toxicity, irritation, allergic response, or other problems or complications, and commensurate with a reasonable benefit/risk ratio.

The term "pharmaceutically acceptable salt" includes salts formed by basic radicals and free acids and salts formed by acidic radicals and free bases, for example, hydrochloride, hydrobromide, nitrate, sulfate, phosphate, formate, acetate, trifluoroacetate, fumarate, oxalate, maleate, citrate, succinate, mesylate, benzenesulfonate, *p*-methylbenzenesulfonate, sodium salt, potassium salt, ammonium salt or amino acid salt, preferably, hydrochloride, hydrobromide, sulfate, formate, acetate, trifluoroacetate, fumarate, maleate, mesylate, p-methylbenzenesulfonate, sodium salt, potassium salt, ammonium salt, and amino acid salt, etc. In the present application, when forming a pharmaceutically acceptable salt, the free acid and the basic radical are in a molar ratio of about 1:0.5 to 1:5, preferably 1:0.5, 1:1, 1:2, 1:3, 1:4, 1:5, 1:6, 1:7 or 1:8. In the present application, when forming a pharmaceutically acceptable salt, the free base and the acidic radical are in a molar weight ratio of about 1:0.5 to 1:5, preferably 1:0.5, 1:1, 1:2, 1:3, 1:4, 1:5, 1:6, 1:7 or 1:8.

The term "fixed combination" refers to administration of the active components (for example, the chemotherapeutic agent or the compound I) to a subject simultaneously at a fixed total dose or in a fixed dose proportion, or in the form of a single substance, pharmaceutical composition or formulation.

The term "non-fixed combination" refers to simultaneous, parallel, or sequential and temporally unlimited administration of two or more aforementioned active components as independent substances (for example, a pharmaceutical composition and a formulation) to a subject, wherein the active ingredients administered to the subject reach therapeutically effective amounts. An example, which can be listed, of the non-fixed combination is a cocktail therapy, for example, 3 or more active components are administered. In a non-fixed combination, each active component can be packaged, sold or administered as a fully independent pharmaceutical composition. The term "non-fixed combination" also includes combined use of "fixed combinations", or a "fixed combination" and an independent substance of any one or more active components.

As used herein, "combined use" or "use in combination" means that two or more active substances may be administered to a subject as a mixture, simultaneously as a single formulation, or sequentially in any order as a single formulation.

The term "pharmaceutical composition" refers to a mixture consisting of one or more of the active ingredients (e.g, the second therapeutic agent or the compound I) or pharmaceutical combinations thereof of the present application and a pharmaceutically acceptable excipient. The pharmaceutical composition is intended to facilitate the administration of the compound or the pharmaceutical combination thereof to a subject.

The "clinical benefits" in the present application include, but are not limited to: prolonged progression-free survival (PFS), prolonged overall survival (OS), improved objective response rate (ORR), improved disease control rate (DCR), reduced number and/or degree of adverse effects, decreased distant metastasis rate, decreased local control rate and the like for clinical patients. In particular, in some specific embodiments of the present invention, especially in specific examples of the present invention, the objective response rate in clinical trials for human patients diagnosed with head and neck cancer is about 10% or more, preferably about 15% or more, further preferably about 20% or more, more preferably about 30% or more, especially 35% or more; the disease control rate of the patients is 50% or more, preferably about 60% or more, more preferably about 70% or more, more preferably about 80% or more, especially 90% or more, at which point the drugs of the present application show clinical benefits.

In the present application, "about" refers to the fluctuation within ±5%, preferably within ±2%, and more preferably within ±1% of the specified numerical range given.

As used herein, unless otherwise stated, the terms "comprise", "comprises" and "comprising" or equivalents thereof are open-ended statements and mean that elements, components and steps that are not specified may be included in addition to those listed.

All patents, patent applications and other identified publications are expressly incorporated herein by reference for the purpose of description and disclosure. These publications are provided solely because they were disclosed prior to the filing date of the present application. All statements as to the dates of these documents or description as to the contents of these documents are based on the information available to the applicant and do not constitute any admission as to the correctness of the dates of these documents or the content of these documents. Moreover, in any country or region, any reference to these publications herein is not to be construed as an admission that the publications form part of the commonly recognized knowledge in the art.

## DETAILED DESCRIPTION

The present application is further described below with reference to specific examples, which are, however, only for illustration and do not limit the scope of the present application. Likewise, the present application is not limited to any specific preferred embodiment described herein. It should be appreciated by those skilled in the art that equivalent substitutions or corresponding modifications for the technical features of the present application still fall with the scope of the present application. Unless otherwise stated, the reagents used in the following examples are commercially available products, and the solutions can be prepared by conventional techniques in the art.

Example 1: Effect of Dihydrochloride of Compound I on Growth of Human Laryngeal Carcinoma Cells Preparation method:

The test drugs were dissolved in dimethyl sulfoxide to prepare a 100 mmol/L stock solution, which was stored at -20 °C for later use. When in use, the stock solution was diluted with a DMEM serum culture medium to a desired concentration.

Cell culturing:
The human laryngeal squamous cell carcinoma Hep-2 cell line and the human laryngeal carcinoma TU212 cell line were separately cultured in a DMEM complete culture medium containing 10% fetal bovine serum and 0.1 g/L streptomycin and penicillin (the final concentration is 100 U•mL$^{-1}$), and the mixtures were incubated in an incubator at 37 °C/5% $CO_2$. When the cell fusion reached about 85%, the cells were mixed and digested using 0.02% EDTA and 0.25% trypsin, and then the cells were collected, centrifuged at 1000 r/min, and subcultured.

Experimental procedures:
The $IC_{50}$ value can be measured by a conventional method in the art (e.g., conventional MTT method), or can be measured by the following method (MTT method).

Cells in logarithmic growth phase were inoculated in a 96-well culture plate (180 (μL/well, $10^5$ cells/well); after 24 h of incubation at 37 °C/5% $CO_2$, the dihydrochloride of the compound I (solutions at gradient concentrations of 0 μg/mL, 0.005 μg/mL, 0.1 μg/mL, 0.05 μg/mL, 0.1 μg/mL, 0.5 μg/mL, 1.5 μg/mL, 4 μg/mL, 12 μg/mL and 30 μg/mL) was added, and the resulting mixtures were incubated. Two duplicate wells were set for each concentration, with 20 μL added to each well. Meanwhile, wells for normal saline solvent controls with corresponding concentrations and cell-free zeroing wells were set. The tumor cells were incubated at 37 °C/5% $CO_2$ for 24 h (i.e., 48 h in total); after the drug effect was finished, an MTT working solution was added into each well, and after 4 h, the triplex solution (SDS-isobutanol-HCl) was added for dissolving. The resulting mixtures were left to stand overnight at 37 °C. The next day, OD values were measured at 570 nm and 630 nm wavelengths using a microplate reader (SPECTRA max 190) (calculated by subtracting OD values measured at 630 nm (the control wavelength) from all OD values measured at 570 nm), and the cell growth inhibition rate was calculated according to the following equation:

$$\text{Inhibition rate} = (\text{OD value of control well} - \text{OD value of administration well})/\text{OD value of control well} \times 100\%$$

Half maximal inhibitory concentration $IC_{50}$ was calculated according to inhibition rates at all concentrations using GraphPad Prism 5 software.

Experimental results:
The *in vitro* pharmacodynamic effect of the dihydrochloride of the compound I on the human laryngeal squamous cell carcinoma Hep-2 cell line and the human laryngeal carcinoma TU212 cell line shows that the dihydrochloride of the compound I has a clear inhibitory effect on the proliferation of the human laryngeal squamous cell carcinoma cells and the human laryngeal carcinoma cells.

Example 2: Effect of Dihydrochloride of Compound I on Growth of Human Oral Squamous Cell Carcinoma Cells Preparation method:
The test drugs were dissolved in dimethyl sulfoxide to prepare a 100 mmol/L stock solution, which was stored at -20 °C for later use. When in use, the stock solution was diluted with a DMEM serum culture medium to a desired concentration.

Cell culturing:
The human oral squamous cell carcinoma scc4 cell line, the human oral squamous cell carcinoma CAL-27 cell line and the human oral squamous cell carcinoma HSC-4 cell line were separately cultured in a DMEM complete culture medium containing 10% fetal bovine serum and 0.1 g/L streptomycin and penicillin (the final concentration is 100 U•mL$^{-1}$), and the mixtures were incubated in an incubator at 37 °C/5% $CO_2$. When the cell confluence reached about 85%, the cells were mixed and digested using 0.02% EDTA and 0.25% trypsin, and then the cells were collected, centrifuged at 1000 r/min, and subcultured.

Experimental procedures:
The $IC_{50}$ value can be measured by a conventional method in the art (e.g., conventional MTT method), or can be measured by the following method (MTT method).

Cells in logarithmic growth phase were inoculated in a 96-well culture plate (180 μL/well, $10^5$ cells/well); after 24 h of incubation at 37 °C/5% $CO_2$, the dihydrochloride of the compound I (solutions at gradient concentrations of 0 μg/mL, 0.005 μg/mL, 0.1 μg/mL, 0.05 μg/mL, 0.1 μg/mL, 0.5 μg/mL, 1.5 μg/mL, 4 μg/mL, 12 μg/mL and 30 μg/mL) was added, and the resulting mixtures were incubated. Two duplicate wells were set for each concentration, with 20 μL added to each well. Meanwhile, wells for normal saline solvent controls with corresponding concentrations and cell-free zeroing wells were set. The tumor cells were incubated at 37 °C/5% $CO_2$ for 24 h (i.e., 48 h in total); after the drug effect was finished, an MTT working solution was added into each well, and after 4 h, the triplex solution (SDS-isobutanol-HCl) was added for dissolving. The resulting mixtures were left to stand overnight at 37 °C. The next day, OD values were measured at 570 nm and 630 nm wavelengths using a microplate reader (SPECTRA max 190) (calculated by subtracting OD values measured at 630 nm (the control wavelength) from all OD values measured at 570 nm), and the cell growth inhibition rate was calculated according to the following equation:

$$\text{Inhibition rate} = (\text{OD value of control well} - \text{OD value of administration well})/\text{OD value of control well} \times 100\%$$

Half maximal inhibitory concentration $IC_{50}$ was calculated according to inhibition rates at all concentrations using GraphPad Prism 5 software.

Experimental results:

The *in vitro* pharmacodynamic effect of the dihydrochloride of the compound I on the human oral squamous cell carcinoma scc4 cell line, the human oral squamous cell carcinoma CAL-27 cell line and the human oral squamous cell carcinoma HSC-4 cell line shows that the dihydrochloride of the compound I has a clear inhibitory effect on the proliferation of the human oral squamous cell carcinoma cells.

Example 3: Clinical Study of Anlotinib Hydrochloride Capsules for Treatment of Recurrent/Metastatic Head and Neck Adenocarcinomas

Patients with histologically or pathologically confirmed head and neck adenocarcinoma that conventional therapy has failed to treat or advanced head and neck adenocarcinoma (including mucoepidermoid carcinoma, adenoid cystic carcinoma, pleomorphic adenocarcinoma, salivary duct carcinoma, myoepithelial carcinoma, non-specific adenocarcinoma, non-specific clear cell carcinoma, cystadenocarcinoma, sebaceous adenocarcinoma, sebaceous lymphoid adenocarcinoma, mucinous adenocarcinoma, and epithelial-myoepithelial carcinoma) were administered with anlotinib hydrochloride capsules once daily, orally before breakfast at a dose of 12 mg each time on d1-14 of each 21-day treatment cycle, with consecutively 2-week treatment and then 1-week interruption. This study showed that, for patients diagnosed with head and neck adenocarcinoma, the administration regimen of anlotinib had clinical benefits.

Example 4

Patients with histologically or pathologically confirmed head and neck squamous cell carcinoma that had local recurrence or distant metastasis and were intolerant to radical therapies (including radiotherapy and surgeries) after previously receiving radiotherapy and/or surgeries, and/or platinum therapy, specifically ones with histopathologically and/or cytologically confirmed squamous cell carcinoma in the oral cavity, oral pharynx, larynx and/or hypopharynx, were co-administered with anlotinib and sintilimab. The administration regimens are detailed below.

Anlotinib hydrochloride capsules: administered once daily, orally before breakfast at a dose of 12 mg each time, on d1-14 of each 21-day treatment cycle, with consecutively 2-week treatment and then 1-week interruption. Sintilimab: 200 mg, i.v. infusion, administered on d1 of each 21-day treatment cycle.

All patients continued for a treatment period of up to 12 months, or until progressive disease, development of an intolerable toxic response, death, or other event requiring discontinuation of treatment specified in the protocol, whichever occurred first.

The efficacy evaluation observation indexes include progression-free survival (PFS), overall survival (OS), objective response rate (ORR = CR + PR), disease control rate (DCR = CR + PR + SD) and drug safety.

This study showed that, for patients diagnosed with head and neck squamous cell carcinoma, the combined administration regimen of anlotinib and sintilimab had clinical benefits.

Patient Case 1:

Patient, male, 55 years old, recurrence after the treatment of laryngeal cancer (recurrence in the parotid gland region), lung metastases. The combined administration regimens of anlotinib and sintilimab are as follows: anlotinib hydrochloride capsules: administered once daily, orally before breakfast, at a dose of 12 mg each time, on d1-14 of each 21-day treatment cycle, with consecutively 2-week treatment and then 1-week interruption; sintilimab: 200 mg, i.v. infusion, administered on d1 of each 21-day treatment cycle. After 4-cycle treatment, the efficacy was evaluated as PR (partial response).

Patient Case 2:

Patient, male, 57 years old, recurrence after the treatment of floor of mouth cancer. The combined administration regimens of anlotinib and sintilimab are as follows: anlotinib hydrochloride capsules: administered once daily, orally before breakfast, at a dose of 12 mg each time, on d1-14 of each 21-day treatment cycle, with consecutively 2-week treatment and then 1-week interruption; sintilimab: 200 mg, i.v. infusion, administered on d1 of each 21-day treatment cycle.

After 8-cycle treatment, the efficacy was evaluated as SD (stable disease).

Example 5

Previously untreated patients with pathologically confirmed head and neck cancer, specifically including ones with nasopharyngeal carcinoma, tonsil cancer, floor of mouth cancer, oral cancer, laryngeal cancer and hypopharyngeal cancer, were all received intensity modulated radiotherapy after administration of anlotinib. The administration regimens are detailed below.

Anlotinib: administered about one week before radiotherapy, 10 mg/day for a three-cycle treatment, consecutively two-week treatment and then one-week interruption, and whether the medicine was administered continuously was determined according to the change in tumors after radiotherapy.

The efficacy evaluation observation indexes include 3-year overall survival (OS), progression-free survival (DFS), 3-year distant metastasis rate and local control rate.

Patient Case 1:

Patient, male, 32 years old, pathologically diagnosed with tonsil cancer, starting taking anlotinib hydrochloride capsules on October 10, 2018 once daily, orally before breakfast at a dose of 10 mg each time, with consecutively two-week treatment and then one-week interruption; starting receiving radiation therapy on October 15, 2018.

| Time of radiotherapy | Dose of radiotherapy (Gy) | Efficacy evaluation |
|---|---|---|
| Week 1 | 10.75 | - |
| Week 2 | 21.5 | - |
| Week 3 | 32.25 | - |
| Week 4 | 43.0 | PR (partial response) |
| Week 5 | 53.75 | - |
| Week 6 | One-week interruption | - |
| Week 7 | 64.5 | - |
| Week 8 | 74.15 | PR (partial response) |

Patient Case 2:

Patient, male, 45 years old, pathologically diagnosed with nasopharyngeal carcinoma, starting taking anlotinib hydrochloride capsules on November 05, 2018 once daily, orally before breakfast at a dose of 10 mg each time, with consecutively two-week treatment and then one-week interruption; starting receiving radiation therapy on November 10, 2018.

| Time of radiotherapy | Dose of radiotherapy (Gy) | Efficacy evaluation |
|---|---|---|
| Week 1 | 10.75 | - |
| Week 2 | 21.5 | - |
| Week 3 | 32.25 | PR (partial response) |
| Week 4 | 43.0 | - |
| Week 5 | 53.75 | - |
| Week 6 | One-week interruption | Neck lump with marked regression, but CR (complete response) not achieved |
| Week 7 | 64.5 | - |
| Week 8 | 74.15 | - |

This study showed that, for patients diagnosed with head and neck cancer, the combined administration regimen of anlotinib and radiotherapy had clinical benefits.

Example 6

Patients with histologically or cytologically confirmed nasopharyngeal carcinoma that had not previously received systemic anti-tumor therapy for recurrent/metastatic nasopharyngeal carcinoma were co-administered with anlotinib, gemcitabine and cisplatin with every 21 days as a cycle. The administration regimens are detailed below.

Administration regimens during combination chemotherapy:

Anlotinib hydrochloride capsules/placebos: administered at an initial dose of 12 mg/0 mg, orally before breakfast, one granule per day, with consecutively two-week treatment and then one-week interruption.

Gemcitabine hydrochloride for injection: administered at an initial dose of 1 g/$m^2$ on days 1 and 8 of each cycle. Cisplatin injection: administered at an initial dose of 75 mg/$m^2$ on day 1 of each cycle.

Administration regimens during maintenance treatment:
Anlotinib hydrochloride capsules/placebos: administered at a dose of 12 mg, orally before breakfast, one granule per day of each 21-day treatment cycle, with consecutively two-week treatment and then one-week interruption, until the termination criteria of the study were reached.

The efficacy evaluation observation indexes include progression-free survival (PFS), overall survival (OS), objective response rate (ORR = CR + PR), disease control rate (DCR = CR + PR + SD) and drug safety.

This study showed that, for patients diagnosed with nasopharyngeal carcinoma, the combined administration regimen of anlotinib, gemcitabine and cisplatin had clinical benefits.

Example 7

Patients with histologically confirmed inoperable, non-radiotherapeutic, recurrent and/or metastatic nasopharyngeal carcinoma that had received platinum chemotherapy or had received at least first-line chemotherapy after recurrence (wherein the inoperable and non-radiotherapeutic nasopharyngeal carcinoma accords with any one of the followings: 1. recurrence after radiotherapy, 2. metastasis, and 3. patients considered unsuitable for surgery and radiotherapy by the relevant multidisciplinary team (MDT) specialists) were co-administered with anlotinib and capecitabine. The administration regimens are detailed below.

Anlotinib hydrochloride capsules: administered once daily, orally before breakfast, at a dose of 12 mg each time, on d1-14 of each 21-day treatment cycle, with consecutively 2-week treatment and then 1-week interruption, and administered continuously until PD, death and intolerable toxicity occurred.

Capecitabine tablets: administered orally at a total dose of 1000 mg/$m^2$, bid, on d1-14 of each 21-day treatment cycle, with consecutively 2-week treatment and then 1 -week interruption, and administered continuously until PD, death and intolerable toxicity occurred.

The efficacy evaluation observation indexes include objective response rate (ORR), progression-free survival (PFS), overall survival (OS), quality of life and drug safety indexes.

This study showed that, for patients diagnosed with nasopharyngeal carcinoma, the combined administration regimen of anlotinib and capecitabine had clinical benefits.

In the above examples of the present application, the amount of anlotinib hydrochloride capsule was by weight of the free base of anlotinib comprised therein.

According to the content disclosed in the present application, the compositions and methods of the present application have been described in terms of preferred embodiments. However, it will be apparent to those skilled in the art that variations may be applied to the compositions and/or methods and the steps or the sequence of steps of the methods described herein without departing from the concept, spirit and scope of the present application.

The disclosed contents of all documents cited herein are hereby incorporated by reference to the extent that they provide exemplary, procedural and other details supplementary to those described herein.

## Claims

1. Use of a compound I or a pharmaceutically acceptable salt thereof for preparing a medicament for use in treating head and neck cancer,

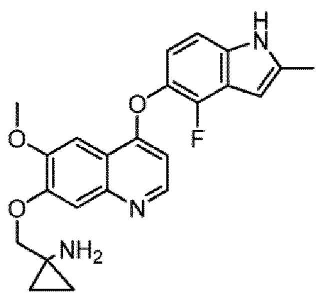

compound I,

wherein the head and neck cancer is selected from the group consisting of oral cancer, laryngeal cancer, parotid gland cancer, nasal cavity cancer, sinus cancer, paranasal sinus cancer, oropharyngeal cancer, floor of mouth cancer, hypopharyngeal cancer, palate cancer, gum cancer, tongue cancer, tongue mucosal squamous cell carcinoma, buccal mucosal carcinoma, lip cancer, salivary gland cancer and tonsil cancer.

2. Use of a compound I or a pharmaceutically acceptable salt thereof in combination with a second therapeutic agent for preparing a medicament for use in treating head and neck cancer, wherein the second therapeutic agent is one or more of a chemotherapeutic agent, a small molecule targeted anti-tumor agent, an immunotherapeutic agent, and a macromolecular antibody drug,

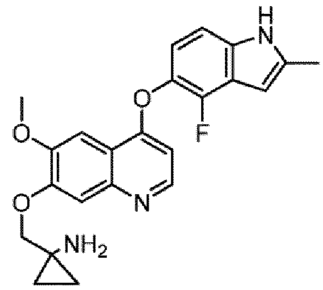

compound I,

wherein the head and neck cancer is selected from the group consisting of oral cancer, laryngeal cancer, parotid gland cancer, thyroid cancer, nasopharyngeal carcinoma, nasal cavity cancer, sinus cancer, paranasal sinus cancer, oropharyngeal cancer, floor of mouth cancer, hypopharyngeal cancer, palate cancer, gum cancer, tongue cancer, tongue mucosal squamous cell carcinoma, buccal mucosal carcinoma, lip cancer, salivary gland cancer and tonsil cancer.

3. The use according to claim 1 or 2, wherein the head and neck cancer is head and neck squamous cell carcinoma or head and neck adenocarcinoma.

4. The use according to claim 3, wherein the head and neck adenocarcinoma is selected from the group consisting of mucoepidermoid carcinoma, adenoid cystic carcinoma, lymphoepithelial carcinoma, pleomorphic adenocarcinoma, salivary duct carcinoma, myoepithelial carcinoma, non-specific adenocarcinoma, non-specific clear cell carcinoma, cystadenocarcinoma, sebaceous adenocarcinoma, sebaceous lymphoid adenocarcinoma, mucinous adenocarcinoma and epithelial-myoepithelial carcinoma.

5. The use according to claim 2, wherein the nasopharyngeal carcinoma is keratinizing squamous cell carcinoma, differentiated non-keratinizing carcinoma, undifferentiated non-keratinizing carcinoma, orthotopic nasopharyngeal carcinoma, squamous cell carcinoma, adenocarcinoma, micro-invasive carcinoma, alveolar cell nuclear carcinoma, or undifferentiated nasopharyngeal carcinoma.

6. The use according to any one of claims 1 to 5, wherein the head and neck cancer is occult cancer or unapparent primary head and neck cancer, locally advanced and/or advanced head and neck cancer, metastatic head and neck cancer, recurrent or unresectable head and neck cancer, or head and neck cancer that chemotherapeutic agents and/or targeted agents have failed to treat.

7. The use according to claim 1 or 2, wherein the head and neck cancer is laryngeal cancer, laryngeal squamous cell carcinoma or oral squamous cell carcinoma.

8. The use according to any one of claims 1 to 7, wherein the compound I or the pharmaceutically acceptable salt thereof is further in combination with radiation therapy.

9. The use according to any one of claims 2 to 7, wherein the chemotherapeutic agent is one or more of oxaliplatin, cisplatin, carboplatin, nedaplatin, dicycloplatin, lobaplatin, triplatin tetranitrate, phenanthriplatin, picoplatin, miriplatin, satraplatin, gemcitabine, capecitabine, ancitabine, fluorouracil, difuradin, doxifluridine,
tegafur, carmofur, trifluridine, paclitaxel, albumin bound paclitaxel and docetaxel, camptothecin, hydroxycamptothecin, 9-aminocamptothecin, 7-ethylcamptothecin, irinotecan, topotecan, vinorelbine, vinblastine, vincristine, vindesine, vinflunine, epirubicin, doxorubicin, daunorubicin, pirarubicin, amrubicin, idarubicin, mitoxantrone, aclarubicin, valrubicin, zorubicin, pixantrone, pemetrexed, carmustine, melphalan, etoposide, teniposide, mitomycin, ifosfamide, cyclophosphamide, azacitidine, methotrexate, bendamustine, liposomal doxorubicin, actinomycin D, bleomycin, pingyangmycin, temozolomide, dacarbazine, peplomycin, eribulin, plinabulin, sapacitabine, treosulfan, 153Sm-EDTMP, tegafur-gimeracil-oteracil potassium and encequidar.

**10.** The use according to any one of claims 2 to 7, wherein the immunotherapeutic agent is one or more of interferon, interleukins, sirolimus, everolimus, ridaforolimus and temsirolimus.

**11.** The use according to any one of claims 2 to 7, wherein the small molecule targeted anti-tumor agent is one or more of imatinib, sunitinib, nilotinib, bosutinib, saracatinib, pazopanib, trabectedin, regorafenib, cediranib, bortezomib, panobinostat, carfilzomib, ixazomib, apatinib, erlotinib, afatinib, crizotinib, ceritinib, vemurafenib, dabrafenib, cabozantinib, gefitinib, dacomitinib, osimertinib, olmutinib, alectinib, brigatinib, lorlatinib, trametinib, larotrectinib, icotinib, lapatinib, vandetanib, selumetinib, sorafenib, olmutinib, savolitinib, fruquintinib, entrectinib, dasatinib, ensartinib, lenvatinib, itacitinib, pyrotinib, binimetinib, erdafitinib, axitinib, neratinib, cobimetinib, acalabrutinib, famitinib, masitinib, ibrutinib, rociletinib, nintedanib, lenalidomide, LOXO-292, vorolanib, bemcentinib, capmatinib, entrectinib, TAK-931, ALT-803, palbociclib, famitinib L-malate, LTT-462, BLU-667, ningetinib, tipifarnib, poziotinib, DS-1205c, capivasertib, SH-1028, DMBG, seliciclib, OSE-2101, APL-101, berzosertib, idelalisib, lerociclib, ceralasertib, PLB-1003, tomivosertib, AST-2818, SKLB-1028, D-0316, LY-3023414, allitinib, MRTX-849, AP-32788, AZD-4205, lifirafenib, vactosertib, mivebresib, napabucasin, sitravatinib, TAS-114, molibresib, CC-223, rivoceranib, CK-101, LXH-254, simotinib, GSK-3368715, TAS-0728, masitinib, tepotinib, HS-10296, AZD-4547, merestinib, olaptesed pegol, galunisertib, ASN-003, gedatolisib, defactinib, lazertinib, CKI-27, S-49076, BPI-9016M, RF-A-089, RMC-4630, AZD-3759, antroquinonol, SAF-189s, AT-101, TTI-101, naputinib, LNP-3794, HH-SCC-244, ASK-120067, CT-707, epitinib succinate, tesevatinib, SPH-1188-11, BPI-15000, copanlisib, niraparib, olaparib, veliparib, talazoparib tosylate, DV-281, siremadlin, telaglenastat, MP-0250, GLG-801, ABTL-0812, bortezomib, tucidinostat, vorinostat, resminostat, epacadostat, tazemetostat, entinostat, mocetinostat, quisinostat, LCL-161, and KML-001.

**12.** The use according to any one of claims 2 to 7, wherein the macromolecular antibody drug is one or more of bevacizumab, ramucirumab, pertuzumab, trastuzumab, cotuximab, nimotuzumab, panitumumab, necitumumab, dinutuzumab, rituximab, temitumumab, ofatumumab, obinutuzumab, alemtuzumab, daratumumab, gemtuzumab, elotuzumab, brentuximab, inotuzumab ozogamicin, blinatumomab, nivolumab, pembrolizumab, durvalumab, toripalimab, sintilimab, camrelizumab, tislelizumab, genolimzumab, lizumab, HLX-10, BAT-1306, AK103, AK104, CS1003, SCT-I10A, F520, SG001, GLS-010, atezolizumab, avelumab, durvalumab, KL-A167, SHR-1316, BGB-333, JS003, STI-A1014, KN035, MSB2311, HLX-20, CS-1001, ipilimumab, tremelimumab, AGEN-1884, BMS-986249, BMS-986218, AK-104, and IBI310.

**13.** The use according to any one of claims 2 to 7, wherein the second therapeutic agent is one or more of a fluoropyrimidine derivative, a platinum agent and an anti-PD-1 antibody.

**14.** The use according to any one of claims 2 to 7, wherein the second therapeutic agent is any one or more of the following (1) to (28):

(1) one, two or three of methotrexate, tegafur-gimeracil-oteracil potassium, and paclitaxel; (2) one or two of a platinum agent and 5-fluorouracil; (3) one or two of a platinum agent and cotuximab; (4) one or two of gemcitabine and vinorelbine; (5) one or two of gemcitabine and paclitaxel; (6) one or two of paclitaxel and carboplatin; (7) one or two of daunorubicin and cytarabine; (8) one or two of mitoxantrone and etoposide; (9) one or two of gemcitabine and cisplatin; (10) one or two of adriamycin and cisplatin; (11) one, two or three of ifosfamide, mesna and etoposide; (12) one, two or three of docetaxel, cisplatin and 5-fluorouracil; (13) one, two or three of cisplatin, epirubicin and paclitaxel; (14) one, two or three of a platinum agent, 5-fluorouracil and cotuximab; (15) one, two or three of a platinum agent, docetaxel and paclitaxel; (16) one, two or three of carboplatin, paclitaxel and gemcitabine; (17) one, two or three of vinorelbine, methotrexate and bleomycin; (18) one, two, three or four of paclitaxel, ifosfamide, mesna and cisplatin; (19) one, two or three of cisplatin, fluorouracil and leucovorin; (20) one, two or three of cisplatin, bleomycin and fluorouracil; (21) one, two or three of mitoxantrone, fluorouracil and carboplatin; (22) one, two or three of pirarubicin, cisplatin and fluorouracil; (23) one, two, three or four of daunorubicin, cytarabine, thioguanine and etoposide; (24) harringtonine, cytarabine and thioguanine; (25) one, two, three or four of harringtonine, vincristine, cytarabine and prednisone; (26) cisplatin and 5-fluorouracil; (27) sillizumab; and (28) capecitabine.

**15.** The use according to any one of claims 1 to 14, wherein the compound I or the pharmaceutically acceptable salt thereof is administered at a daily dose of 3 mg to 30 mg, preferably 5 mg to 20 mg, more preferably 8 mg to 16 mg, further more preferably 8 mg to 14 mg, and most preferably 8 mg, 10 mg, or 12 mg.

**16.** The pharmaceutical composition according to any one of claims 1 to 15, wherein the compound I or the pharmaceutically acceptable salt thereof is administered on days 1-14 of each 21-day cycle.

**INTERNATIONAL SEARCH REPORT**

International application No. **PCT/CN2020/092125**

**A. CLASSIFICATION OF SUBJECT MATTER**

A61K 31/4709(2006.01)i； A61P 35/00(2006.01)i； A61P 35/04(2006.01)i

According to International Patent Classification (IPC) or to both national classification and IPC

**B. FIELDS SEARCHED**

Minimum documentation searched (classification system followed by classification symbols)

A61K; A61P

Documentation searched other than minimum documentation to the extent that such documents are included in the fields searched

Electronic data base consulted during the international search (name of data base and, where practicable, search terms used)

CNPAT, CNKI, WPI, SIPOABS, CAPLUS, REGISTRY, PUBMED 安罗替尼, AL3818, Anlotinib, 1058156-90-3, 头颈, 头, 颈, 口腔, 喉, 腮腺, 鼻腔, 鼻, 鼻窦, 副鼻窦, 口咽, 口底, 下咽, 腭, 牙龈, 舌, 舌粘膜, 颊粘膜, 唇, 唾液腺, 扁桃体, 甲状腺, 鼻咽, 癌, 肿瘤, 腺癌, 鳞癌, head and neck, head, neck, oral cavity, larynx, parotid gland, nasal cavity, nose, sinuses, paranasal sinuses, oropharynx, floor of mouth, hypopharynx, palate, gums, tongue, tongue mucosa, buccal mucosa, lips, salivary glands, tonsils, thyroid, nasopharyngeal, tumor, tumour, cancer, oncoma, neoplas+, antitumor, anticancer, antineoplas+, carcinoma, adenocarcinoma, squamous cell carcinoma

**C. DOCUMENTS CONSIDERED TO BE RELEVANT**

| Category* | Citation of document, with indication, where appropriate, of the relevant passages | Relevant to claim No. |
|---|---|---|
| X | CN 105213394 A (CHIA TAI TIANQING PHARMACEUTICAL GROUP CO., LTD.) 06 January 2016 (2016-01-06)<br>claims 7-9 | 1, 6, 8, 15, 16 |
| X | CN 107771078 A (ADVENCHEN PHARMACEUTICALS LLC; CHEN GUOQING PAUL) 06 March 2018 (2018-03-06)<br>claims 8-16, 19-23 | 1-16 |

☐ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **06 August 2020** | **31 August 2020** |

| Name and mailing address of the ISA/CN | Authorized officer |
|---|---|
| **China National Intellectual Property Administration (ISA/CN)**<br>**No. 6, Xitucheng Road, Jimenqiao Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2020/092125**

| Patent document cited in search report | Publication date (day/month/year) | Patent family member(s) | Publication date (day/month/year) |
|---|---|---|---|
| CN 105213394 A | 06 January 2016 | CN 106413712 B | 16 August 2019 |
| | | CN 105213394 B | 09 April 2019 |
| | | CN 106413712 A | 15 February 2017 |
| | | CN 111012785 A | 17 April 2020 |
| | | WO 2015185011 A1 | 10 December 2015 |
| | | CN 111035640 A | 21 April 2020 |
| | | KR 20170007789 A | 20 January 2017 |
| CN 107771078 A | 06 March 2018 | US 2016326138 A1 | 10 November 2016 |
| | | US 2019002435 A1 | 03 January 2019 |
| | | MX 2017014108 A | 16 March 2018 |
| | | CN 110292579 A | 01 October 2019 |
| | | EP 3291814 A1 | 14 March 2018 |
| | | CN 110292580 A | 01 October 2019 |
| | | CA 2984444 A1 | 10 November 2016 |
| | | US 10100034 B2 | 16 October 2018 |
| | | AU 2016257816 A1 | 14 December 2017 |
| | | KR 20180014716 A | 09 February 2018 |
| | | US 10544125 B2 | 28 January 2020 |
| | | EP 3291814 A4 | 05 December 2018 |
| | | WO 2016179123 A1 | 10 November 2016 |
| | | CN 110294741 A | 01 October 2019 |
| | | US 9751859 B2 | 05 September 2017 |
| | | TW 201639830 A | 16 November 2016 |
| | | BR 112017023639 A2 | 17 July 2018 |
| | | US 2018002311 A1 | 04 January 2018 |
| | | EA 201792355 A1 | 29 June 2018 |
| | | CN 110172057 A | 27 August 2019 |
| | | JP 2018515482 A | 14 June 2018 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 105026428 B
- WO 2015085847 A1